# EUROPEAN PATENT SPECIFICATION

(11) **EP 2 861 271 B1**
(45) Date of publication and mention of the grant of the patent: **19.09.2018**
(21) Application number: 13714445.7
(22) Date of filing: 18.03.2013
(51) Int. Cl.: A61L 31/06, A61L 31/14, C08L 67/04

(54) **BIORESORBABLE POLYMER PERIPHERAL SCAFFOLDS MADE FROM BLOCK COPOLYMERS OF POLY(L-LACTIDE) AND HYDROPHILIC POLYMERS**
BIORESORBIEBARE PERIPHERE POLYMERGERÜSTE AUS BLOCKCOPOLYMEREN AUS POLY(L-LACTID) UND HYDROPHILEN POLYMEREN
ÉCHAFAUDAGES PÉRIPHÉRIQUES EN POLYMÈRE BIORÉSORBABLE RÉALISÉS À PARTIR DE COPOLYMÈRES BLOCS DE POLY(L-LACTIDE) ET DE POLYMÈRES HYDROPHILES

(30) Priority: 15.06.2012 US 201213525145
(43) Date of publication of application: 22.04.2015
(73) Proprietor: Abbott Cardiovascular Systems Inc., Santa Clara, CA 95054 (US)
(72) Inventor: JAYASINGHE, Dudley S., Murrieta, California 92562 (US); DAVALIAN, Dariush, San Jose, California 95124 (US); FARNBACH, Ronald A., Temecula, California 92592 (US)
(74) Representative: ZBM Patents - Zea, Barlocci & Markvardsen
(86) International application number: PCT/US2013/032849
(87) International publication number: WO 2013/187988

(56) References cited:
- WO-A1-2011/094621
- WO-A1-2013/052183
- WO-A2-2009/045808
- US-A1- 2011 190 872
- US-A1- 2012 073 733
- VENKATRAMAN S S ET AL: "Biodegradable stents with elastic memory", BIOMATERIALS, ELSEVIER SCIENCE PUBLISHERS BV., BARKING, GB, vol. 27, no. 8, 1 March 2006 (2006-03-01), pages 1573-1578, XP027950913, ISSN: 0142-9612 [retrieved on 2006-03-01]

## Description

### BACKGROUND OF THE INVENTION

### Field of the Invention

This invention relates polymeric medical devices, in particular, bioresorbable stents or stent scaffoldings.

### Description of the State of the Art

This invention relates to radially expandable endoprostheses that are adapted to be implanted in a bodily lumen. An "endoprosthesis" corresponds to an artificial device that is placed inside the body. A "lumen" refers to a cavity of a tubular organ such as a blood vessel. A stent is an example of such an endoprosthesis. Stents are generally cylindrically shaped devices that function to hold open and sometimes expand a segment of a blood vessel or other anatomical lumen such as urinary tracts and bile ducts. Stents are often used in the treatment of atherosclerotic stenosis in blood vessels. "Stenosis" refers to a narrowing or constriction of a bodily passage or orifice. In such treatments, stents reinforce body vessels and prevent restenosis following angioplasty in the vascular system. "Restenosis" refers to the reoccurrence of stenosis in a blood vessel or heart valve after it has been treated (as by balloon angioplasty, stenting, or valvuloplasty) with apparent success.

Stents are typically composed of a scaffold or scaffolding that includes a pattern or network of interconnecting structural elements or struts, formed from wires, tubes, or sheets of material rolled into a cylindrical shape. This scaffolding gets its name because it physically holds open and, if desired, expands the wall of the passageway. Typically, stents are capable of being compressed or crimped onto a catheter so that they can be delivered to and deployed at a treatment site.

Delivery includes inserting the stent through small lumens using a catheter and transporting it to the treatment site. Deployment includes expanding the stent to a larger diameter once it is at the desired location. Mechanical intervention with stents has reduced the rate of restenosis as compared to balloon angioplasty. Yet, restenosis remains a significant problem. When restenosis does occur in the stented segment, its treatment can be challenging, as clinical options are more limited than for those lesions that were treated solely with a balloon.

Stents are used not only for mechanical intervention but also as vehicles for providing biological therapy. Biological therapy uses medicated stents to locally administer a therapeutic substance. A medicated stent may be fabricated by coating the surface of either a metallic or polymeric scaffold with a polymeric carrier that includes an active or bioactive agent or drug. Polymeric scaffolds may also serve as a carrier of an active agent or drug. An active agent or drug may also be included on a scaffold without being incorporated into a polymeric carrier.

The stent must be able to satisfy a number of mechanical requirements. The stent must be capable of withstanding the structural loads, namely radial compressive forces, imposed on the scaffold as it supports the walls of a vessel. Therefore, a stent must possess adequate radial strength. Radial strength, which is the ability of a stent to resist radial compressive forces, relates to a stent's radial yield strength and radial stiffness around a circumferential direction of the stent. A stent's "radial yield strength" or "radial strength" (for purposes of this application) may be understood as the compressive loading, which if exceeded, creates a yield stress condition resulting in the stent diameter not returning to its unloaded diameter, i.e., there is irrecoverable deformation of the stent. When the radial yield strength is exceeded the stent is expected to yield more severely and only a minimal force is required to cause major deformation. Radial strength is measured either by applying a compressive load to a stent between flat plates or by applying an inwardly-directed radial load to the stent.

Once expanded, the stent must adequately maintain its size and shape throughout its service life despite the various forces that may come to bear on it, including the cyclic loading induced by the beating heart. For example, a radially directed force may tend to cause a stent to recoil inward. In addition, the stent must possess sufficient flexibility to allow for crimping, expansion, and cyclic loading.

Some treatments with stents require its presence for only a limited period of time. Once treatment is complete, which may include structural tissue support and/or drug delivery, it may be desirable for the stent to be removed or disappear from the treatment location. One way of having a stent disappear may be by fabricating a stent in whole or in part from materials that erodes or disintegrate through exposure to conditions within the body. Stents fabricated from biodegradable, bioabsorbable, bioresorbable, and/or bioerodable materials such as bioabsorbable polymers can be designed to completely erode only after the clinical need for them has ended.

The development of a bioresorbable stent or scaffold could obviate the permanent metal implant in vessel, allow late expansive luminal and vessel remodeling, and leave only healed native vessel tissue after the full absorption of the scaffold. A fully bioresorbable stent can reduce or eliminate the risk of potential long-term complications and of late thrombosis, facilitate non-invasive diagnostic MRI /CT imaging, allow restoration of normal vasomotion, and provide the potential for plaque regression.

To treat peripheral vascular disease percutaneously in the lower limbs is a challenge with current technologies. Long term results are sub-optimal due to chronic injury caused by the constant motions of the vessel and the implant as part of every day life situations. To reduce the chronic injury a bioresorbable scaffold for the superficial femoral artery (SFA) and/or the popliteal artery can be used so that the scaffold disappears before it causes any significant long term damage. However, one of the challenges with the development of a femoral scaffold and especially a longer length scaffold (5 - 25 cm) to be exposed to the distal femoral artery and potentially the popliteal artery is the presence of fatigue motions that may lead to chronic recoil and strut fractures especially in the superficial femoral artery, prior to the intended bioresorption time especially when implanted in the superficial femoral artery.
A scaffold in the SFA and/or the popliteal artery is subjected to various nonpulsatile forces, such as radial compression, torsion, flexion, and axial extension and compression. These forces place a high demand on the scaffold mechanical performance and can make the scaffold more susceptible to fracture than less demanding anatomies. In addition to high radial strength, stents or scaffolds for peripheral vessels such as the SFA, require a high degree of crush recovery. The term "crush recovery" is used to describe how the scaffold recovers from a pinch or crush load, while the term "crush resistance" is used to describe the force required to cause a permanent deformation of a scaffold.
Particularly, the United States patent application US2012073733 discloses a process for preparing peripherical stent having crush recoverable polymer scaffolds, wherein the stent comprises a scaffold crimped to a balloon-catheter. The crush-recoverable polymer scaffold has greater than about 80% crush recoverability when crushed to about 50% of its starting diameter for longer duration crush periods; and wherein the radial yield strength of the balloon expanded polymer scaffold has radial yield strength of greater than about 0.3 N/mm, and the polymer is a block copolymer. Further, the stents comprising a scaffold with a network of interconnected elements corresponding to pattern 200 of sequence of crowns: W-crown, free crown, Y-crown, free crown; or alternatively, the stents comprising a scaffold with a network of interconnected elements corresponding to pattern 300. That pattern has the following sequence of crowns: W-crown, free crown, Y-crown, 3 free crown and W-crown.
The International patent application WO2009046808 discloses a biodegradable stent comprising a scaffold fabricated at least in part from a polymer composite including a biodegradable elastomeric phase dispersed within a biodegradable polymeric matrix. This composite is formed by block copolymers including an elastomeric homopolymer block and a glassy polymer block.
Therefore, an important goal for treatment of the SFA and/or the popliteal artery is the development of bioabsorbable scaffold with high radial strength, high crush recovery, and high resistance to fracture or high toughness.

The present invention provides a stent comprising a scaffold formed from a polymer tube - configured for being crimped to a balloon, - the scaffold having a pattern of interconnected elements, - the scaffold having an expanded diameter when expanded from a crimped state by the balloon, wherein the scaffold attains greater than about 80% of its diameter after being crushed to at least 50% of its expanded diameter; wherein the scaffold has a radial stiffness greater than 0.3 N/mm and wherein the scaffold is made from a block copolymer of poly(L-lactide) (PLLA) and a hydrophilic polymer capable of having self expanding properties at 37 deg C; and wherein the scaffold has rings formed by struts, and the struts form undulating rings having crowns and the rings are interconnected by longitudinally extending links, wherein the crowns include W-crowns, Y-crowns, and free crowns, wherein a link connects a W-crown of a ring to a first adjacent ring and another link connects a Y-crown of the ring to a second adjacent ring, wherein a free crown is not connected to a link; and wherein the rings comprise a sequence of crowns including: W-crown, 3 free crowns, Y-crown, 3 free crowns, and W-crown.

### BRIEF DESCRIPTION OF THE DRAWINGS

FIG. 1 depicts a first embodiment of a crush recoverable scaffold pattern.
FIG. 2 is a partial perspective view of a scaffold structure.
FIG. 3 depicts a second embodiment of a crush-recoverable scaffold structure.
FIG. 4A depicts aspects of the repeating pattern of closed cell elements associated with the pattern of FIG. 3.
FIG. 4B depicts aspects of the repeating pattern of closed cell elements associated with the pattern of FIG. 1.
FIGS. 5A and 5B are tables showing examples of scaffold features in accordance with aspects of the disclosure.
FIG. 6 depicts a third embodiment of a crush-recoverable scaffold structure.
FIG. 7 depicts aspects of the repeating pattern of closed cell elements associated with the pattern of FIG. 6.
FIG. 8A shows the radial strength and stiffness for PLLA V79 and PLLA-PEG V79 scaffolds.
FIG. 8B shows the radial strength and stiffness for several samples of PLLA V59 and PLLA-PEG V79 scaffolds. FIG. 9 shows the radial strength and stiffness for V79 PLLA and V79 PLLA-PCL scaffold samples.
FIG. 10 depicts crush recovery results for two lots of V59 PLLA scaffolds, a V79 PLLA scaffold, and a V79 PLLA-PEG scaffold.
FIG. 11 depicts the number of cycles of an axial fatigue test vs. the number of fracture for V59 PLLA and V79 PLLA-PEG scaffolds.
FIG. 12 shows discontinuity count in ring and connector links after axial fatigue testing for the PLLA, 95:5 PLLA-PCL, and 90:10 PLLA-PCL scaffolds.
FIG. 13 shows discontinuity percentage in ring and connector links after axial fatigue testing for the V79 PLLA, 95:5 PLLA-PCL, and 90:10 PLLA-PCL scaffolds.
FIGs. 14-16 depict Finescan images of the V59 PLLA scaffold and the V79 PLLA and V79 PLLA-PEG scaffolds, respectively, post deployment.

### DETAILED DESCRIPTION OF THE INVENTION

The invention is defined by the appended claims.

The embodiments described herein are generally applicable to polymeric implantable medical devices, especially those that have load bearing portions when in use or have portions that undergo deformation during use. In particular, the methods can be applied to tubular implantable medical devices such as self-expandable stents, balloon-expandable stents, and stent-grafts.

A stent or scaffold may include a tubular scaffold structure that is composed of a plurality of ring struts and link struts. The ring struts form a plurality of cylindrical rings arranged about the cylindrical axis. The rings are connected by the link struts. The scaffold comprises an open framework of struts and links that define a generally tubular body with gaps in the body defined by the rings and struts. A thin-walled cylindrical tube of may be formed into this open framework of struts and links described by a laser cutting device that cuts such a pattern into the thin-walled tube that may initially have no gaps in the tube wall.

The scaffold may also be fabricated from a sheet by rolling and bonding the sheet to form the tube. A tube or sheet can be formed by extrusion or injection molding. The scaffold can then be crimped on to a balloon or catheter for delivery into a bodily lumen.

A stent or scaffold of the present invention can be made partially or completely from a biodegradable, bioresorbable, and bioabsorbable polymer. The stent can also be made in part of a biostable polymer. A polymer for use in fabricating stent can be biostable, bioresorbable, bioabsorbable, biodegradable or bioerodable. Biostable refers to polymers that are not biodegradable. The terms biodegradable, bioresorbable, bioabsorbable, and bioerodable are used interchangeably and refer to polymers that are capable of being completely degraded and/or eroded into different degrees of molecular levels when exposed to bodily fluids such as blood and can be gradually resorbed, absorbed, and/or eliminated by the body. The processes of breaking down and absorption of the polymer can be caused by, for example, hydrolysis and metabolic processes.

Bioresorbable stents or scaffolds can be useful for treatment of various types of bodily lumens including the coronary artery, superficial femoral artery, popliteal artery, neural vessels, and the sinuses. In general, these treatments require the stent to provide mechanical support to the vessel for a period of time and then desirably to absorb away and disappear from the implant site. The important properties of a bioresorbable stent or scaffolding include mechanical and degradation properties. The mechanical requirements include high radial strength, high radial stiffness, and high fracture toughness. The degradation properties include the absorption profile, for example, the change in molecular weight, radial strength, and mass with time. Specific aspects of the absorption profile include the time that the stent maintains radial strength before starting to decrease and the total absorption time or absorption time (complete mass loss from implant site).

A stent scaffolding made from a bioresorbable polymer may be designed to maintain its radial strength and/or radial stiffness once implanted to provide mechanical support to the vessel for a prescribed time period and maintain patency of the lumen. The radial strength must be sufficiently high initially to support the lumen at a desired diameter. The period of time that the scaffold is required or desired to maintain patency depends on the type of treatment, for coronary treatment it is about 3 months. After this time period, the vessel is healed sufficiently to maintain an expanded diameter without support. Therefore, after this time period, the scaffolding may start to lose radial strength and/or radial stiffness due to molecular weight degradation. As the scaffolding degrades further, it starts to lose mechanical integrity and then experiences mass loss and eventually absorbs away completely or there are negligible traces left behind.

Ideally, it is desired that once the stent support is no longer needed by the lumen, the bioresorbable scaffold should be resorbed as fast as possible while also meeting all basic safety requirements during its degradation period. Such safety requirements can include a gradual disintegration and resorpton that does not allow release of fragments that could cause adverse events such as thrombosis. In this way, the stent scaffold enables the vessel healing as well as enabling the advantages mentioned herein of a bioresorbable scaffold to the greatest extent. It is desirable for a bioresorbable scaffold to have an absorption time of about 18 to 26 months for coronary vascular application, of about eighteen months (e.g., 16-20 months) for a peripheral application (e.g., superficial femoral artery (SFA)) and/or popliteal artery), 18-24 months for neural applications, and less than a year for nasal applications.

With respect to radial strength and stiffness, a stent should have sufficient radial strength and/or stiffness to withstand structural loads, namely radial compressive forces, imposed on the stent so that the stent can supports the walls of a vessel at a selected target diameter for a desired time period. A polymeric stent with adequate radial strength and/or stiffness enables the stent to maintain a lumen at a desired diameter for a sufficient period of time after implantation into a vessel.

In addition, the stent should possess sufficient toughness or resistance to fracture to allow for crimping, expansion, and cyclic loading without fracture or cracking that would compromise the function of the stent. The toughness or resistance to fracture of the scaffold material can be characterized for a material by the elongation at break and for a stent by the number and degree of cracks in a scaffold during use, such as after crimping or deployment or dilation to a target diameter. These aspects of the use of the scaffold involve deformation of various hinge portions of the structural elements of the scaffold.

Some bioresorbable polymers, for example, semi-crystalline polymers, are stiff or rigid under physiological conditions within a human body and have been shown to be promising for use as a scaffold material. Physiological conditions include a temperature at or about 37 deg C and exposure to bodily fluids, in particular, a wet or aqueous environment. Specifically, polymers that have a glass transition temperature (Tg) sufficiently above human body temperature which is approximately 37 °C, should be stiff or rigid upon implantation. Poly(L-lactide) (PLLA) is attractive as a stent material due to its relatively high strength and a rigidity at human body temperature, about 37 °C. PLLA has high strength and tensile modulus compared to other biodegradable polymers. Since it has a glass transition temperature well above human body temperature, it remains stiff and rigid at human body temperature. This property facilitates the ability of a PLLA stent scaffolding to maintain a lumen at or near a deployed diameter.

Other rigid bioresorbable polymers include poly(D-lactide) (PDLA), polyglycolide (PGA), and poly(L-lactide-co-glycolide) (PLGA). The PLGA include those having a mole% of (LA:GA) of 85:15 (or a range of 82:18 to 88:12), 95:5 (or a range of 93:7 to 97:3), or commercially available PLGA products identified being 85:15 or 95:5 PLGA. Rigid polymers may refer to polymers that have a Tg higher than human body temperature or within 5 deg C of human body temperature.

The strength, stiffness, and the fracture toughness of such polymers can be improved through various processing methods (e.g., radial expansion and suitable choice of associated processing parameters). However, there is still strong incentive to improve upon polymers such as PLLA as scaffold materials not only for coronary applications, but to tailor it for other peripheral applications as well. In particular, such polymers may be improved upon to reduce chronic recoil inward from a deployed diameter and to reduce strut fractures due to fatigue motions imposed on scaffolds implanted in such vessels as the popliteal artery and in the superficial femoral artery.

Embodiments of the present invention include bioresorbable scaffolds with reduced risk of scaffold fracture and improved recoil property as compared to PLLA scaffolds. The scaffolds are composed of block copolymers of PLLA and a hydrophilic polymer capable of having self expanding properties at 37 deg C. The block copolymer scaffolds have improved fracture resistance over PLLA scaffolds. The composition of the block copolymers is mostly PLLA and a small weight fraction of hydrophilic polymer. The hydrophilic blocks provide increased fracture toughness through phase separation and/or plasticizing the PLLA.

The hydrophilic polymers can be bioresorbable, water soluble, gel forming, or any combination thereof. The hydrophilic polymers may be non-ionic. Hydrophilic polymers include polyethylene oxide (PEO), polyethylene glycol (PEG), polyvinylpyrrolidone (PVP), polyhydroxybutyrate, polyhydroxyalkanoates, and polyvinyl alcohol (PVA).

Hydrophilic polymers such as PEG and PVA tend to swell due to uptake of water upon exposure to moist environments, such as in a physiological environment. When exposed to an aqueous environment or bodily fluids, the block copolymers may include 1 to 20 wt% of water, or more narrowly, 2 to 10%, or 5 to 8% of water. Therefore, the mechanical properties of the scaffold in wet (post implant) and dry (pre-implant) conditions can be quite different. The swelling of the block copolymer decreases the modulus and increases the fracture resistance of the polymer. Therefore, the radial stiffness of the scaffold decreases and the fracture resistance of the scaffold increases. Additionally, the Tg of the block copolymer decreases due to the swelling which results in shape memory behavior of the scaffold.

The hydrophilic blocks can phase separate from the PLLA and form domains distributed or dispersed through the PLLA. Without being limited by theory, the polymer domains may induce energy dissipation in the more stiff PLLA and retard crack initiation and propagation to improve fracture toughness. The characteristic length (diameter, length, width) of domains can be 250 nm to 5 um, or more narrowly 250 nm to 1 µm, 100 nm to 500 nm, 500 nm to 1 µm, 1 µm to 2 µm, 2 µm to 5 µm, or greater than 5 µm.

In other embodiments, hydrophilic domains are not formed or observed. However, without being limited by theory, in the absence of domains, the hydrophilic polymer may decrease stiffness and increase fracture toughness of the PLLA through plasticizing of the PLLA. Plasticizing refers generally to increasing the plasticity of a material. Plasticity or plastic deformation describes the deformation of a polymer undergoing non-reversible changes of shape in response to applied forces. A hydrophilic polymer that plasticizes PLLA increases its flexibility and toughness, thereby, increasing resistance to fracture. Without being limited by theory, a plasticizer works by embedding between the chains of a stiff polymer like PLLA, spacing them apart (increasing the "free volume"), and thus significantly lowering the glass transition temperature of the PLLA and making it softer.

The Tg reduction, percent water uptake, and microphase separation depend in part on the fraction of hydrophilic polymer and the size or molecular weight of the hydrophilic blocks. The Tg of the block copolymer decreases as the hydrophilic polymer fraction increases. The percent water uptake increases as the hydrophilic polymer fraction and block size increase. The tendency for microphase separation of the hydrophilic blocks increases as the hydrophilic polymer fraction and block size increase.

The hydrophilic block may be 0.5 to 1%, 1 to 5%, 1 to 10%, 2 to 5%, 3-5 %, 5 to 10%, 5 or about 5%, 10 or about 10% (by weight or molar) of the block copolymer of the scaffold. In other embodiments, the hydrophilic blocks may be 5 to 15% or 15 to 25% of the block copolymer of the scaffold.

The molecular weight of the hydrophilic blocks for any of the ranges or values of fractions and any of the types of block copolymers may be 1 to 30 kDa, or more narrowly, 1 to 10 kDa, 2 to 4 kDa, 4 to 10 kDa, or 6 to 10 kDa. The molecular weight of the hydrophilic blocks for any of the ranges or values of fractions can also be 20 to 100 kDa, or more narrowly, 50 to 100 kDa, or 60 to 100 kDa.

The molecular weight of the PLLA blocks for any of the values or ranges of molecular weights and compositions of the hydrophilic blocks can be 50 to 300 kDa, or more narrowly, 70 to 100 kDa, 100 to 200 kDa, or 200 to 300 kDa,

The block copolymers can include linear block copolymers such as diblock (AB), triblock (ABA), or, generally, multiblock copolymers (ABABA). ABA triblock polymers can have A as a PLLA block and B as the hydrophilic block (e.g., PEG). Exemplary block copolymers include PLLA-b-PEG, PLLA-b-PEG-b-PLLA; and PEG-b-PLLA-b-PEG.

The block copolymers can also include star-block and branched copolymers. Star block copolymers can include at least three arms, the arms corresponding to polymer chains. The blocks at the core of the star blocks may be hydrophilic blocks and the outer blocks may be PLLA.

A scaffold can be composed completely or almost completely of a block copolymer including PLLA and hydrophilic blocks. In some embodiments, the shape memory copolymer is not blended with another polymer. The scaffold, however, may include small amounts of other additives such as antioxidants, inorganic reinforcing agents, or therapeutic agents, for example, 0.1 to 2 wt%.

Another embodiment is a blend of the block copolymer and another polymer. The scaffold may further include a polymer and drug coating.

In an alternate embodiment, a PLLA scaffold may have a coating of the block copolymer. The PLLA scaffold can be dip or spray coated with the block copolymer, for example PLLA-b-PEG. The shape memory coating may reduce or prevent PLLA crack propagation and strut separation prior to tissue coverage. The coating may be 2 to 20 microns.

As described above, the block copolymer scaffolds may exhibit shape memory properties in physiological conditions. Shape-memory properties refer to the ability of a material to return from a deformed state (temporary shape) to an original (permanent) shape induced by an external stimulus (trigger), such as a temperature change or thermal transition. A scaffold of the present invention has a tendency return or self expand towards a fabricated diameter when deployed from a crimped or reduced diameter at physiological conditions.

The temperature of the thermal transition of a shape memory polymer is referred to as a transition temperature (Ttrans) which is the temperature around which a material changes from one state to another. In general, a Ttrans can be either a melting temperature (Tm) or glass transition temperature (Tg). In the present invention, the Tg of the block copolymer of the scaffold is the relevant thermal transition that induces shape memory behavior of an implanted scaffold. Ttrans is typically determined by differential scanning calorimetry (DSC), thermomechanical analysis (TMA) or dynamic mechanical thermal analysis (DMA). DSC measures the change in heat capacity, TMA measures the change in coefficient of thermal expansion, while DMA measures the change in elastic modulus during the thermal transition. Due to intrinsic polydispersity in molecular weights and imperfect spatial distribution of network chains, the unique thermal characteristics of a polymer should be defined as a temperature range rather than at one specific temperature. For the ease of comparison, however, a single Ttrans (Tm or Tg) value taken from the peak or midpoint of a broader transition is often reported in literature.

As described herein, the block copolymer of the of the scaffold is composed mostly of poly(L-lactide) and a small amount of hydrophilic polymer, both in the form of blocks of the block copolymer. The small amount of the hydrophilic polymer together with the poly(L-lactide) exhibit shape memory behavior upon deployment in a vessel at physiological conditions.

The block copolymer scaffolds may be fabricated from a block copolymer tubing at a given diameter and crimped down to a reduced diameter over a catheter balloon. The crimped scaffold is deployed from the reduced diameter to a target or nominal expansion diameter in a vessel. Although the scaffold has a tendency to self expand, the expansion is assisted by the catheter balloon. The scaffold may be further fabricated so that it exhibits an outward force at the target diameter by oversizing (i.e., as-fabricated diameter greater than the target diameter) the scaffold relative to the target diameter. The outward force is a residual force in excess of a radial outward force that maintains the lumen at the target diameter. The oversizing is advantageous due to the increased flexibility or lower modulus of the shape memory and reinforcing scaffold.

The oversized scaffold is made from a tube with a diameter (e.g., 8 to 10 mm) greater than the target diameter (6 to 8 mm). The tube may be oversized by expanding the tubing greater than the intended target diameter. The expansion also results in sufficient scaffold radial strength and stiffness to treat a stenosed artery at early duration of the implant. Due to the oversizing, the deployed scaffold applies an outward force on the vessel walls at the target diameter which can a result in expansion of the vessel to a diameter slightly greater than the target diameter. The outward force is analogous to the chronic outward force applied by self-expanding metallic stents such as nitinol stents.

At physiological conditions of 37 deg C and hydration in blood, the bioresorbable block copolymer exhibits a thermal transition which results in shape memory behavior that includes a tendency for the scaffold to self-expand, to exert an outward residual force on the vessel wall, or both. The shape memory behavior, however, acts in a time-dependent fashion. In contrast to permanent nitinol stents, the self-reinforcing bioresorbable scaffold will degrade, reducing its residual outward force with time, transform from independent load-bearing member into tissue-incorporated composite, and ultimately disappear without causing any significant chronic vascular injury.

The mechanism of controlled reduction in scaffolding property (radial strength/stiffness/strain recovery) will reduce tissue compliance (vascular compliance in case of a vascular implant) mismatch of the implanted segment. Specifically, the scaffold undergoes degradation in three phases.

In the first phase, the molecular weight is reduced primarily due to hydrolytic degradation of the polymer chains. During this first phase the mechanical properties such as radial strength and radial stiffness change very little or not at all so that the scaffold supports the vessel at the target diameter. This allows vessel wall remodeling that strengthens it to enable the vessel to support itself at the remodeled diameter once the scaffold is gone. The water content of the scaffold due to swelling accelerates the hydrolytic degradation.

In the second phase, mechanical properties including the radial strength and any residual outward force of the scaffold decrease. The decrease in radial strength in particular results in the transfer of the load or support of the vessel wall from the scaffold to the vessel wall. Also, in the second phase, the scaffold begins to break up due to the deterioration of mechanical properties. Prior to breaking up, the scaffold is preferably covered by tissue. Water soluble hydrophilic polymer blocks leach out of the scaffold during the first and second phases, decreasing the fraction of hydrophilic blocks.

In the third phase, which may overlap with the second phase, the scaffold erodes, eventually completely away from the implant site. From implantation through the third phase the vessel compliance increases gradually from that of the scaffold to that of the natural compliance of an un-scaffolded vessel at a remodeled, increased diameter.

For a PLLA scaffold, the increase in compliance occurs principally through breaking up of the scaffold. However, the compliance of the block copolymer scaffold increases significantly through swelling of the block copolymer, even without fracture of the scaffold.

The residual outward force of the scaffold is lower than that of the chronic outward force of a self expanding nitinol scaffold and will therefore create less injury than the nitinol implant. Additionally, the residual outward force is temporary and eventually disappears. Moreover, the axial and radial flexibility provided by the disclosed structure of the scaffold will reduce even the acute injury that would otherwise potentially occur even before any of either the chemical or mechanical degradation starts.

A PLLA scaffold has a Tg of about 60-65 deg C at dry conditions, and therefore, tends not to exhibit shape memory properties upon expansion in a vessel. Specifically, the PLLA scaffold is expanded to a target diameter, but does not exhibit a tendency to self expand at physiological conditions and also will not apply a residual force in excess of that for maintaining patency of the vessel. The large difference between the Tg of the scaffold in physiological conditions (e.g., 37 deg C and wet) is responsible for the absence of a thermal transition that would provide the tendency to self expand or a residual force.

The hydrophilic polymer may have a Tg in dry or wet conditions less than PLLA in dry or wet conditions, respectively. Therefore, the block copolymer of the scaffold of the present invention may have a glass transition temperature (Tg), particularly in the wet state, that is lower than a PLLA scaffold.

PEG has a Tg of ∼22.37 deg C, therefore, the Tg of a block copolymer is likely less than PLLA in both the dry and the wet physiological state. Some hydrophilic polymers such as PVA and PVP have Tg's in the dry state that are higher than PLLA, ∼85 deg C and ∼175 deg C, respectively. However, the block copolymer in the wet physiological state swells due to the hydrophilic blocks which reduces the Tg of the block copolymer.

The reduction in the Tg of the scaffold closer to or below the physiological conditions of 37 deg C contributes to the shape memory property upon expansion in a vessel to a target diameter. The scaffold copolymer Tg is sufficiently close to the physiological temperature for the scaffold to exhibit a tendency to self expand towards an original fabricated diameter. This tendency reduces or eliminates recoil of the scaffold. If the scaffold is oversized, then the scaffold applies a residual outward force on the vessel wall in excess of that required to maintain the target diameter of the vessel. The residual force further reduces or eliminates recoil of the scaffold.

The Tg of the block copolymer of the scaffold in dry or wet conditions may be 5 to 10 deg C, 10 to 20 deg C, 10 to 30 deg C, 20 to 30 deg C less than the Tg of the PLLA scaffold. Alternatively, the Tg of the block copolymer scaffold may be 30 to 37 deg C, 37 to 40 deg C, 37 to 50 deg C, 40 to 45 deg C, 45 to 50 deg C, or 50 to 55 deg C. The hydrophilic polymer, as a homopolymer, may have a Tg less than 37 deg C, less than 25 deg C, 0 to 25 deg C, or -70 to 0 deg C.

As indicated above, the thermal transition of the shape memory copolymer can occur around, not necessarily only at a thermal transition Trans, for example, Tg. Therefore, the block copolymer of the scaffold need not have a Tg at this temperature for the scaffold to exhibit shape memory properties. The thermal transition may have varying degrees of broadness, i.e., the thermal transition may occur within a certain ΔT below the Tg of the copolymer. The copolymer can be designed to have a selected broadness of both Tg and Tm. For example, a copolymer may be have a broad Tg and a sharp Tm for a shape memory scaffold with a Tg > 37 deg C. A broad Tg or Tm may be defined as a ΔT of between 5 and 20 deg C. A narrow Tg or Tm may be defined as a ΔT of between 5 and 10 deg C. Alternatively, a copolymer may have a sharp Tm and sharp Tg for a shape memory scaffold with Tg < 37 deg C.

A shape memory polymer may also exhibit more than one Tg which may occur in in block copolymers having blocks of sufficiently high molecular weight, for example greater than 100 kDa.

Another advantage of the block copolymer scaffold of the present invention is that physical aging that causes embrittlement of the scaffold polymer occurs over a shorter time frame than for a PLLA scaffold. In general, after fabrication, a polymer of a scaffold undergoes a process called densification that occurs over a period of time until the properties stabilize. The time it takes for the polymer to stabilize is higher for higher Tg's. At the lower Tg of the shape memory copolymer, the physical aging process is faster and stabilizes faster. For example, for a Tg between 40 and 50 deg C, the polymer stabilizes between 0 and 60 days. As a result, the products used for implantation will have high consistency of properties.

Stent scaffold patterns made from PLLA for SFA applications have been designed which have high crush recovery and crush resistance. Crush recovery describes the recovery of a scaffold subjected to a pinch or crush load. Specifically, the crush recovery can be described as the percent recovery to the scaffold pre-crush shape or diameter from a certain percent crushed shape or diameter. Crush resistance is the minimum force required to cause a permanent deformation of a scaffold.

The crush recoverable PLLA scaffolds are disclosed in US2011/0190872 and US2011/0190871 (WO2011094621) . These scaffolds attain greater than about 80% of its diameter after being crushed to at least 50% of its expanded diameter. The scaffolds also have a radial stiffness greater than 0.3 N/mm. Such scaffolds also have a normalized radial strength of at least 0.4 N/mm. The inventors have found a scaffold for use in peripheral application should have these minimum values of radial stiffness and radial strength.

Embodiments further include bioresorbable scaffolds that in addition to high crush recovery, the selected radial stiffness and radial strength, also possess high resistance to fracture and self expanding properties that reduce or eliminate recoil upon deployment. These embodiments include the previously disclosed crush recoverable scaffolds and additional crush recoverable scaffolds disclosed herein made from the shape memory block copolymer disclosed herein. The inventors have found that a suitable combination of scaffold and shape memory block copolymer can result in a scaffold having desired performance characteristics.

The scaffolds of the present invention for peripheral (SFA) applications usually have lengths of between about 36 and 40 mm or even between 40 and 200 mm when implanted in the superficial femoral artery, as an example. The scaffold for the SFA applications may have a pre-crimping diameter of between 5-10 mm, or more narrowly, 6-8 mm. The scaffold for SFA may have a wall thickness of 0.2032 mm to 0.355 mm (0.008" to 0.014") and configured for being deployed by a non-compliant or semi-compliant balloon, e.g., 6.5 mm diameter, from about a 1.8 to 2.2 mm diameter (e.g., 2 mm) crimped profile. The SFA scaffold may be deployed to a diameter of between about 4 mm and 10 mm, or more narrowly, 7 to 9 mm.

These crush recoverable block copolymer scaffolds can attain greater than about 80% of their diameter after being crushed to at least 50% of its expanded diameter. Additionally, such crush recoverable block copolymer scaffolds have a normalized radial strength, as measured by techniques described herein and in cited applications, of greater than about 0.3 N/mm, or between about 0.3 and 1.2 N/mm or between about 0.3 and 1.2 N/mm, and a radial stiffness of greater than about 0.3 N/mm or between about 0.3 and 2 N/mm.

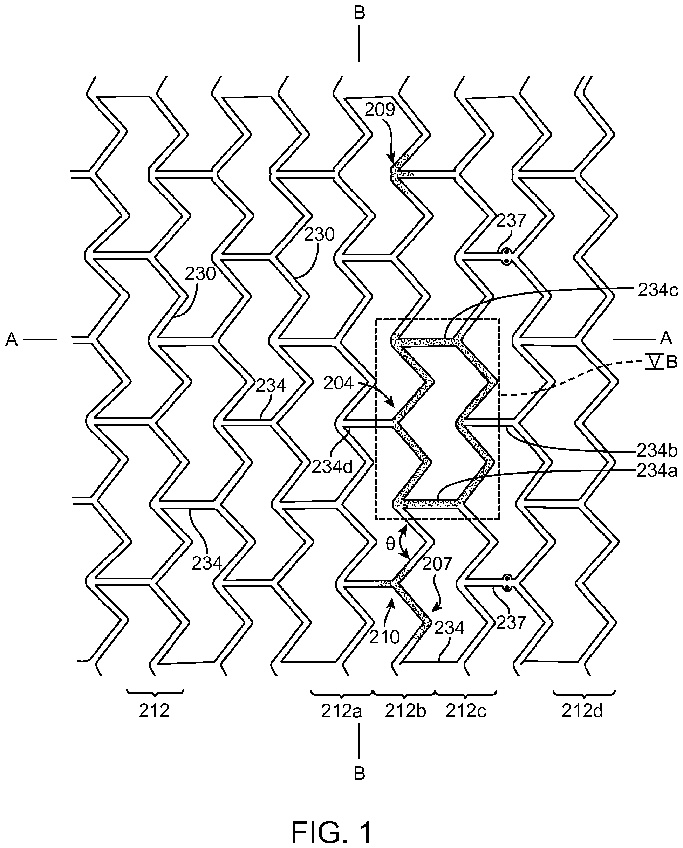

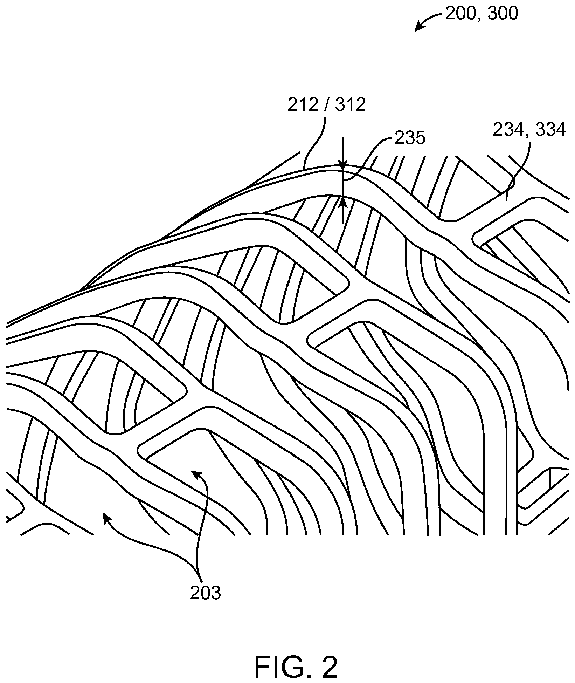

A first embodiment of a crush recoverable scaffold pattern is depicted in FIG. 1. FIG. 1 depicts the pattern 200 (not forming part of the invention) which includes longitudinally-spaced rings 212 formed by struts 230. The pattern 200 of FIG. 1, represents a tubular scaffold structure (as partially shown in three dimensional space in FIG. 2), so that an axis A-A is parallel to the central or longitudinal axis of the scaffold. FIG. 2 shows the scaffold in a state prior to crimping or after deployment. As can be seen from FIG. 2, the scaffold comprises an open framework of struts and links that define a generally tubular body. A cylindrical tube of may be formed into this open framework of struts and links described in FIG. 1.

In FIG. 1, a ring 212 is connected to an adjacent ring by several links 234, each of which extends parallel to axis A-A. In this first embodiment of a scaffold pattern (pattern 200) four links 234 connect the interior ring 212, which refers to a ring having a ring to its left and right in FIG. 1, to each of the two adjacent rings. Thus, ring 212b is connected by four links 234 to ring 212c and four links 234 to ring 212a. Ring 212d is an end ring connected to only the ring to its left in FIG. 1.

A ring 212 is formed by struts 230 connected at crowns 207, 209 and 210. A link 234 is joined with struts 230 at a crown 209 (W-crown) and at a crown 210 (Y-crown). A crown 207 (free-crown) does not have a link 234 connected to it. Preferably the struts 230 that extend from a crown 207, 209 and 210 at a constant angle from the crown center, i.e., the rings 212 are approximately zig-zag in shape, as opposed to sinusoidal for pattern 200, although in other embodiments a ring having curved struts is contemplated. As such, in this embodiment a ring 212 height, which is the longitudinal distance between adjacent crowns 207 and 209/210 may be derived from the lengths of the two struts 230 connecting at the crown and a crown angle θ. In some embodiments the angle θ at different crowns will vary, depending on whether a link 234 is connected to a free or unconnected crown, W-crown or Y-crown.

The zig-zag variation of the rings 212 occurs primarily about the circumference of the scaffold (i.e., along direction B-B in FIG. 1). The struts 212 centroidal axes lie primarily at about the same radial distance from the scaffold's longitudinal axis. Ideally, substantially all relative movement among struts forming rings also occurs axially, but not radially, during crimping and deployment. Although, as explained in greater detail, below, polymer scaffolds often times do not deform in this manner due to misalignments and/or uneven radial loads being applied.

The rings 212 are capable of being collapsed to a smaller diameter during crimping and expanded to a larger diameter during deployment in a vessel. According to one aspect of the disclosure, the pre-crimp diameter (e.g., the diameter of the axially and radially expanded tube from which the scaffold is cut) is always greater than a maximum expanded scaffold diameter that the delivery balloon can, or is capable of producing when inflated. According to one embodiment, a pre-crimp diameter is greater than the scaffold expanded diameter, even when the delivery balloon is hyper-inflated, or inflated beyond its maximum use diameter for the balloon-catheter.

Pattern 200 includes four links 237 (two at each end, only one end shown in FIG. 1) having structure formed to receive a radiopaque material in each of a pair of transversely-spaced holes formed by the link 237. These links are constructed in such a manner as to avoid interfering with the folding of struts over the link during crimping, which, as explained in greater detail below, is necessary for a scaffold capable of being crimped to a diameter of about at most Dmin or for a scaffold that when crimped has virtually no space available for a radiopaque marker-holding structure.

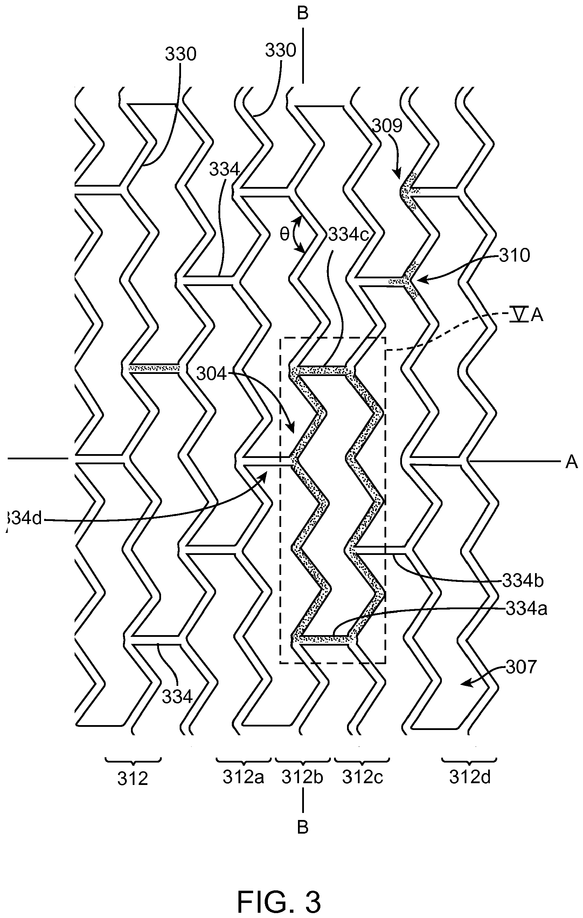

A second embodiment of a crush-recoverable scaffold structure has the pattern 300 (not forming part of the invention) illustrated in FIG. 3. Like the pattern 200, the pattern 300 includes longitudinally-spaced rings 312 formed by struts 330. A ring 312 is connected to an adjacent ring by several links 334, each of which extends parallel to axis A-A. The description of the structure associated with rings 212, struts 230, links 234, and crowns 207, 209, 210 in connection with FIG. 1, above, also applies to the respective rings 312, struts 330, links 334 and crowns 307, 309 and 310 of the second embodiment, except that in the second embodiment there are only three struts 334 connecting each adjacent pair of rings, rather than four. Thus, in the second embodiment the ring 312b is connected to the ring 312c by only three links 234 and to the ring 312a by only three links 334. A link formed to receive a radiopaque marker, similar to link 237, may be included between 312c and ring 312d.

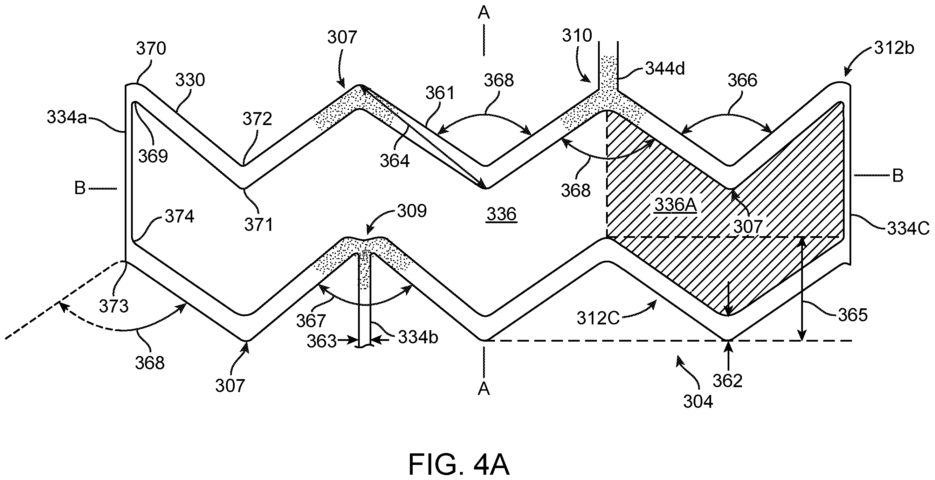

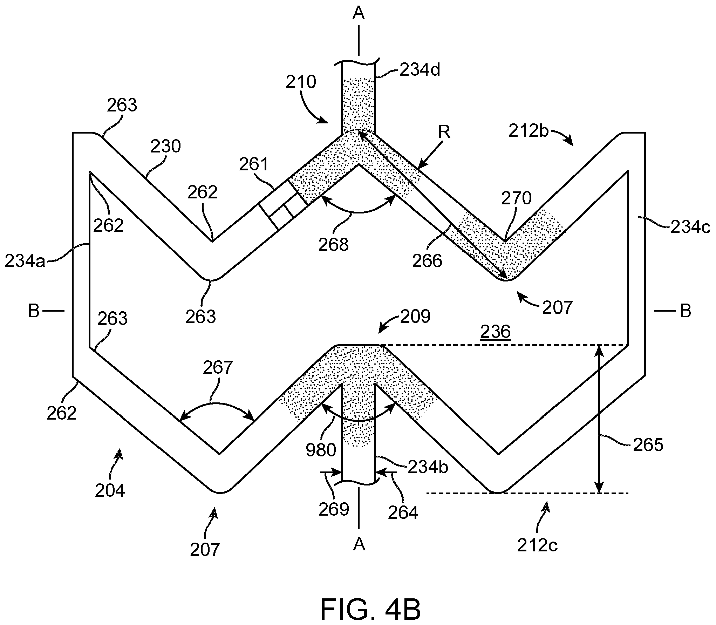

FIGS. 4A and 4B depict aspects of the repeating pattern of closed cell elements associated with each of the patterns 300 and 200, respectively. FIG. 4A shows the portion of pattern 300 bounded by the phantom box VA in FIG. 3 and FIG. 4B shows the portion of pattern 200 bounded by the phantom box VB in FIG. 1. Therein are shown cell 304 and cell 204, respectively. In FIGS. 4A, 4B the vertical axis reference is indicated by the axis B-B and the longitudinal axis A-A. There are four cells 204 formed by each pair of rings 212 in pattern 200, e.g., four cells 204 are formed by rings 212b and 212c and the links 234 connecting this ring pair, another four cells 204 are formed by rings 212a and 212b and the links connecting this ring pair, etc. In contrast, there are three cells 304 formed by a ring pair and their connecting links in pattern 300.

Referring to FIG. 4A, the space 336 and 336a of cell 304 is bounded by the longitudinally spaced rings 312b and 312c portions shown, and the circumferentially spaced and parallel links 334a and 334c connecting rings 312b and 312c. Links 334b and 334d connect the cell 304 to the right and left adjacent ring in FIG. 2, respectively. Link 334b connects to cell 304 at a W-crown 309. Link 334d connects to cell 304 at a Y-crown 310. A "W-crown" refers to a crown where the angle extending between a strut 330 and the link 336 at the crown 310 is an obtuse angle (greater than 90 degrees). A "Y-crown" refers to a crown where the angle extending between a strut 330 and the link 336 at the crown 309 is an acute angle (less than 90 degrees). The same definitions for Y-crown and W-crown also apply to the cell 204. There are eight connected or free crowns 307 for cell 304, which may be understood as eight crowns devoid of a link 334 connected at the crown. There are one or three free crowns between a Y-crown and W-crown for the cell 304.

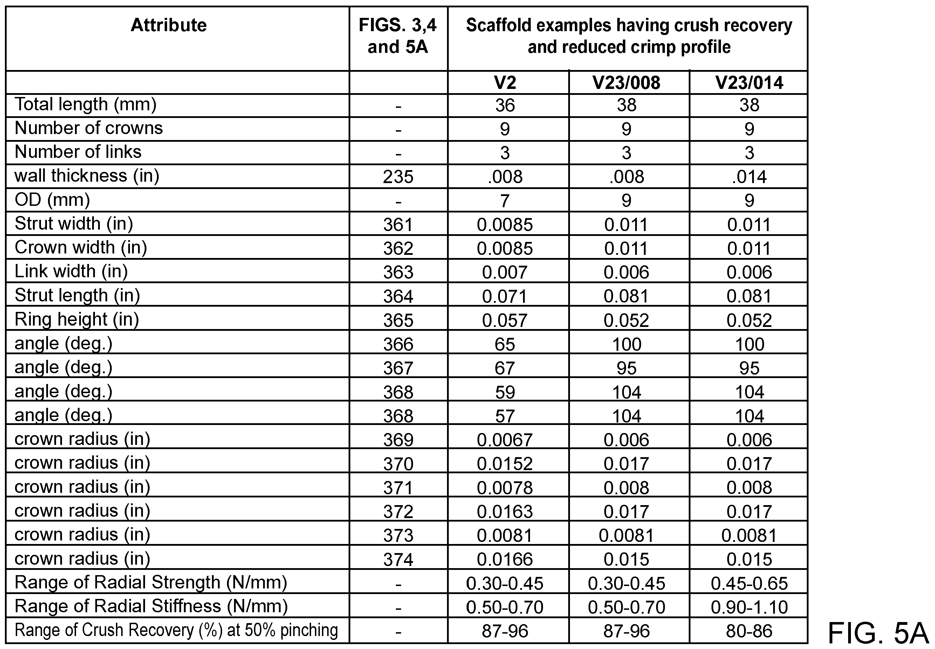

Additional aspects of the cell 304 of FIG. 4A include angles for the respective crowns 307, 309 and 310. Those angles, which are in general not equal to each other (see e.g., FIG. 5A for the "V2" and "V23" embodiments of scaffold having the pattern 300), are identified in FIG. 4A as angles 366, 367 and 368, respectively associated with crowns 307, 309 and 310. For the scaffold having the pattern 300 the struts 330 have strut widths 361 and strut lengths 364, the crowns 307, 309, 310 have crown widths 362, and the links 334 have link widths 363. Each of the rings 312 has a ring height 365. The radii at the crowns are, in general, not equal to each other. The radii of the crowns are identified in FIG. 4A as radii 369, 370, 371, 372, 373 and 374.

Cell 304 may be thought of as a W-V closed cell element. The "V" portion refers to the shaded area 336a that resembles the letter "V" in FIG. 5A. The remaining unshaded portion 336, i.e., the "W" portion, resembles the letter "W".

Referring to FIG. 4B, the space 236 of cell 204 is bounded by the portions of longitudinally spaced rings 212b and 212c as shown, and the circumferentially spaced and parallel links 234a and 234c connecting these rings. Links 234b and 234d connect the cell 204 to the right and left adjacent rings in FIG. 1, respectively. Link 234b connects to cell 236 at a W-crown 209. Link 234d connects to cell 236 at a Y-crown 210. There are four crowns 207 for cell 204, which may be understood as four crowns devoid of a link 234 connected at the crown. There is only one free crown between each Y-crown and W-crown for the cell 204.

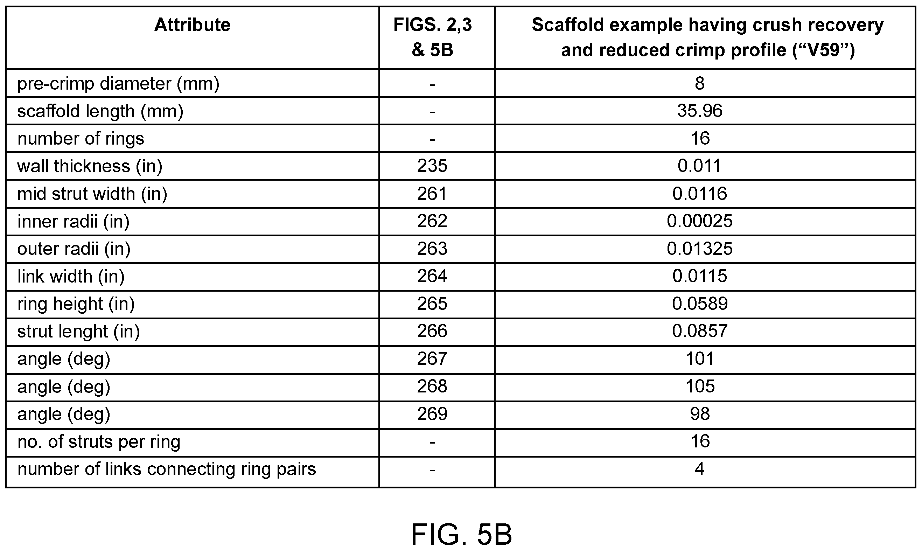

Additional aspects of the cell 204 of FIG. 4B include angles for the respective crowns 207, 209 and 210. Those angles, which are in general not equal to each other (see e.g., FIG. 5B for the "V59" embodiment of a scaffold having the pattern 200), are identified in FIG. 4B as angles 267, 269 and 268, respectively associated with crowns 207, 209 and 210. For the scaffold having the pattern 200 the struts 230 have strut widths 261 and strut lengths 266, the crowns 207, 209, 210 have crown widths 270, and the links 234 have link widths 261. Each of the rings 212 has a ring height 265. The radii at the crowns are, in general, not equal to each other. The radii of the crowns are identified in FIG. 4A as inner radii 262 and outer radii 263.

Cell 204 may be thought of as a W closed cell element. The space 236 bounded by the cell 204 resembles the letter "W".

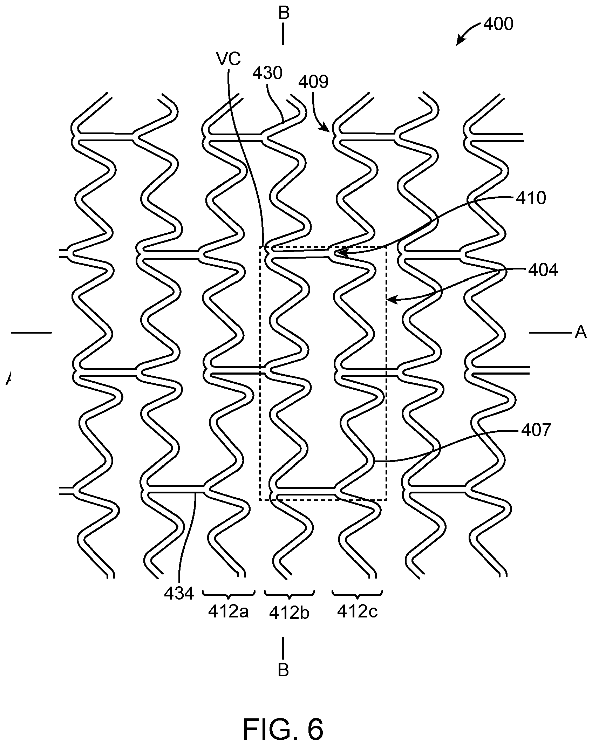

Comparing FIG. 4A to FIG. 4B one can appreciate that the W cell 204 is symmetric about the axes B-B and A-A whereas the W-V cell 304 is asymmetric about both of these axes. The W cell 204 is characterized as having no more than one crown 207 between links 234. Thus, a Y-crown crown or W-crown is always between each crown 207 for each closed cell of pattern 200. In this sense, pattern 200 may be understood as having repeating closed cell patterns, each having no more than one crown that is not supported by a link 234. In contrast, the W-V cell 304 has three unsupported crowns 307 between a W-crown and a Y-crown. As can be appreciated from FIG. 4A, there are three unsupported crowns 307 to the left of link 334d and three unsupported crowns 307 to the right of link 334b.
A third embodiment of a crush-recoverable scaffold structure (referred to herein as "V79") has the pattern 400 illustrated in FIG. 6. Pattern 400 is an image of a portion of a scaffold post deployment which was generated the Visicon Finescan™ Stent Inspection System, and is discussed in detail in the Examples. Therefore, there is apparent a certain amount of distortion in the angles between the various struts. Like the patterns 200 and 300, the pattern 400 includes longitudinally-spaced rings 412 formed by struts 430. A ring 412 is connected to an adjacent ring by links 434, each of which extends parallel to axis A-A. The description of the structure associated with rings 212, struts 230, links 234, and crowns 207, 209, 210 in connection with FIG. 1, above, also applies to the respective rings 412, struts 430, links 434 and crowns 407, 409 and 410 of the third embodiment, except that in the third embodiment there are only two struts 434 connecting each adjacent pair of rings, rather than four. Thus, in the third embodiment the ring 412b is connected to the ring 412c by only two links 434 and to the ring 412a by only two links 434.
The sequence of crests starting at a W-crown and going around a circumference of a ring is: W-crown, 3-free crowns, Y-crown, 3 free crowns, W-crown, etc. The pattern shown in FIG. 6 has 2 W-crowns and 2-Y crowns. Thus, there are 3 free crowns between a W-crown and Y-crown. A link formed to receive a radiopaque marker, similar to link 237, may be included between two rings.

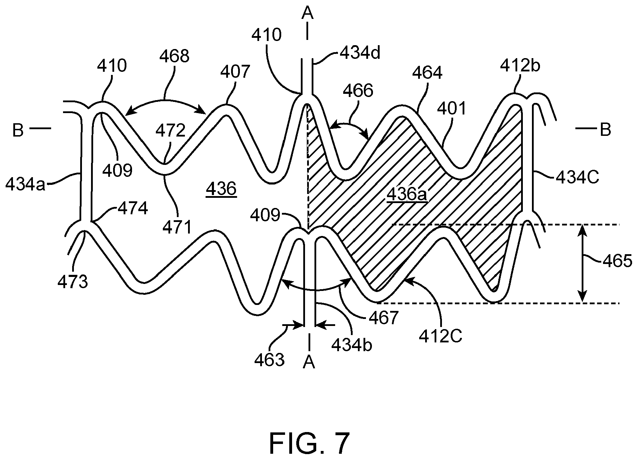

FIG. 7 shows the portion of pattern 400 bounded by the phantom box VC.
Therein are shown cell 404. In FIG. 7, the vertical axis reference is indicated by the axis B-B and the longitudinal axis A-A. There are two cells 404 formed by each pair of rings 412 in pattern 400, e.g., two cells 404 are formed by rings 412b and 412c and the links 434 connecting this ring pair, another two cells 404 are formed by rings 412a and 412b and the links connecting this ring pair, etc.
Referring to FIG. 7, the space 436 and 436a of cell 404 is bounded by the longitudinally spaced rings 412b and 412c portions shown, and the circumferentially spaced and parallel links 434a and 434c connecting rings 412b and 412c. Links 434b and 434d connect the cell 404 to the right and left adjacent ring in FIG. 6, respectively. Link 434b connects to cell 404 at a W-crown 409. Link 434d connects to cell 404 at a Y-crown 410. There are 12 unconnected or free crowns 407 for cell 404, which may be understood as 12 crowns devoid of a link 434 connected at the crown. There are three free crowns between a Y-crown and W-crown for the cell 404.
Additional aspects of the cell 404 of FIG. 7 include angles for the respective crowns 407, 409 and 410. Those angles, which are in general not equal to each other (see e.g., FIG. 5A for the "V2" and "V23" embodiments of scaffold having the pattern 300), are identified in FIG. 7 as angles 466, 467 and 468, respectively associated with crowns 407, 409 and 410. For the scaffold having the pattern 400 the struts 430 have strut widths 461 and strut lengths 464, the crowns 407, 409, 410 have crown widths 462, and the links 434 have link widths 463. Each of the rings 412 has a ring height 465. The radii at the crowns are, in general, not equal to each other. The radii of the crowns are identified in FIG. 7 as radii 469, 470, 471, 472, 473 and 474.

Cell 404 may be thought of as a W-W closed cell element. The unshaded area 436 and unshaded area 436a each resemble the letter "W".

The stent scaffolds may be formed by extruding polymer tubes made of the block copolymer and laser cutting the tubes to form a scaffold. The fabrication methods of bioabsorbable scaffolds described herein can include the following steps:
(1) forming a polymeric tube using extrusion,
(2) radially deforming the formed tube,
(3) forming a stent scaffolding from the deformed tube by laser machining a stent pattern in the deformed tube with laser cutting,
(4) optionally forming a therapeutic coating over the scaffolding,
(5) crimping the stent over a delivery balloon, and
(6) sterilization with election-beam (E-Beam) radiation.

In the extrusion step, a polymer is processed in an extruder above the melting temperature of the copolymer.

In step (2) above, the extruded tube may be radially deformed to increase the radial strength of the tube, and thus, the finished stent. The increase in strength reduces the thickness of the struts required to support a lumen with the scaffold when expanded at an implant site. In exemplary embodiments, the strut thickness can be 100-200 microns, or more narrowly, 120-180, 140-160, or 160-200 microns.

Detailed discussion of the manufacturing process of a bioabsorbable stent can be found elsewhere, e.g., U.S. Patent Publication Nos. 20070283552 and 20120073733. "Molecular weight refers to either number average molecular weight (Mn) or weight average molecular weight (Mw). References to molecular weight herein refer to either Mn or Mw, unless otherwise specified.

"Semi-crystalline polymer" refers to a polymer that has or can have regions of crystalline molecular structure and amorphous regions. The crystalline regions may be referred to as crystallites or spherulites which can be dispersed or embedded within amorphous regions.

The "glass transition temperature," Tg, is the temperature at which the amorphous domains of a polymer change from a brittle vitreous state to a solid deformable or ductile state at atmospheric pressure. In other words, the Tg corresponds to the temperature where the onset of segmental motion in the chains of the polymer occurs. When an amorphous or semi-crystalline polymer is exposed to an increasing temperature, the coefficient of expansion and the heat capacity of the polymer both increase as the temperature is raised, indicating increased molecular motion. As the temperature is increased, the heat capacity increases. The increasing heat capacity corresponds to an increase in heat dissipation through movement. Tg of a given polymer can be dependent on the heating rate and can be influenced by the thermal history of the polymer as well as its degree of crystallinity. Furthermore, the chemical structure of the polymer heavily influences the glass transition by affecting mobility.

The Tg can be determined as the approximate midpoint of a temperature range over which the glass transition takes place. [ASTM D883-90]. The most frequently used definition of Tg uses the energy release on heating in differential scanning calorimetry (DSC). As used herein, the Tg refers to a glass transition temperature as measured by differential scanning calorimetry (DSC) at a 20 °C/min heating rate.

The "melting temperature" (Tm) is the temperature at which a material changes from solid to liquid state. In polymers, Tm is the peak temperature at which a semicrystalline phase melts into an amorphous state. Such a melting process usually takes place within a relative narrow range (<20 °C), thus it is acceptable to report Tm as a single value.

"Stress" refers to force per unit area, as in the force acting through a small area within a plane. Stress can be divided into components, normal and parallel to the plane, called normal stress and shear stress, respectively. Tensile stress, for example, is a normal component of stress applied that leads to expansion (increase in length). In addition, compressive stress is a normal component of stress applied to materials resulting in their compaction (decrease in length). Stress may result in deformation of a material, which refers to a change in length. "Expansion" or "compression" may be defined as the increase or decrease in length of a sample of material when the sample is subjected to stress.

"Strain" refers to the amount of expansion or compression that occurs in a material at a given stress or load. Strain may be expressed as a fraction or percentage of the original length, i.e., the change in length divided by the original length. Strain, therefore, is positive for expansion and negative for compression.

"Strength" refers to the maximum stress along an axis which a material will withstand prior to fracture. The ultimate strength is calculated from the maximum load applied during the test divided by the original cross-sectional area.

"Modulus" may be defined as the ratio of a component of stress or force per unit area applied to a material divided by the strain along an axis of applied force that results from the applied force. The modulus typically is the initial slope of a stress - strain curve at low strain in the linear region. For example, a material has both a tensile and a compressive modulus.

The tensile stress on a material may be increased until it reaches a "tensile strength" which refers to the maximum tensile stress which a material will withstand prior to fracture. The ultimate tensile strength is calculated from the maximum load applied during a test divided by the original cross-sectional area. Similarly, "compressive strength" is the capacity of a material to withstand axially directed pushing forces. When the limit of compressive strength is reached, a material is crushed.

"Toughness" is the amount of energy absorbed prior to fracture, or equivalently, the amount of work required to fracture a material. One measure of toughness is the area under a stress-strain curve from zero strain to the strain at fracture. The units of toughness in this case are in energy per unit volume of material. See, e.g., L. H. Van Vlack, "Elements of Materials Science and Engineering," pp. 270-271, Addison-Wesley (Reading, PA, 1989).

### Examples

Scaffold samples made from a tri-block copolymer were prepared, PLLA-b-PEG-b-PLLA with a composition 90.4/9.6 (w/w%) PLLA/PEG. PLLA (not forming part of the invention) and poly(L-lactide-co-caprolactone) (95:5 and 90:10) (not forming part of the invention) were prepared and tested for comparison. Two lots of the PLLA scaffolds were prepared. The samples were tested after e-beam sterilization. Scaffolds with a poly(DL-lactide)/Everolimus coating were also tested.

### Processing:

90:10 PLLA-PEG copolymer was processed by extrusion (temperature approximately 365 deg F) to form a tube with 0.059 in ID. PLLA tubes were also prepared with using a 450 deg F extrusion temperature. The tubes were expanded to 7mm OD at a temperature above their Tg, but lower than Tm. The PEG-PLLA polymer tube was then laser cut into a V79 scaffold pattern. PLLA polymer tube was then laser cut into a V59 scaffold pattern.

90:10 PLLA-PCL with instrinsic viscosity (IV) of 3.2 was processed by extrusion (water content 310ppm, temp approximately 380 deg F) to form a tube with 0.059 in ID. The tube was expanded to 8mm OD at a temperature above its Tg but lower than Tm. It was then laser cut into a V79 scaffold pattern. L-lactide monomer content in extruded tubing and expanded tubing was 0.12%.

95:5 PLLA-PCL with IV of 3.8 was processed by extrusion (water content 320ppm, temp approximately 370 deg F) to form a tube with 0.059 in ID. The tube was then expanded to 7mm OD at a temperature above its Tg but lower than Tm. It was then laser cut into a V79 scaffold pattern. L-lactide monomer content in extruded tubing and expanded tubing was 0.11%.

The scaffold samples were crimped onto a folded Omnilink Elite PTA balloons (Abbott Vascular Inc., Santa Clara, CA) at 48 deg C, packaged, and sterilized by e-beam.

### Glass Transition Temperature

The glass transition temperature of the PLLA-PEG block copolymer and the PLLA were measured using DSC analysis. The PLLA-PEG block copolymer has Tg of 46 deg C compared to approximately 61 deg C for PLLA. The reduced Tg provides increased flexibility and contributes to the self expansion nature of the triblock copolymer.

### Radial Strength and Stiffness

Radial strength and stiffness were measured on an MSI tester (Machines Solutions Inc., Flagstaff, AZ). The radial strength for all scaffolds was greater than 0.4 N/mm. The radial strength and stiffness of the PLLA and PLLA-PEG V79 scaffolds, both sterile and uncoated, are compared in Table 1.

**Table 1. Radial stiffness and strength of V79 PLLA and PLLA-PEG scaffolds.**

| Scaffold | Radial Stiffness (N/mm) | Radial Strength (N/mm) |
|---|---|---|
| V79PLLA | 1.46 | 0.78 |
| V79 PLLA-PEG | 0.87 | 0.48 |

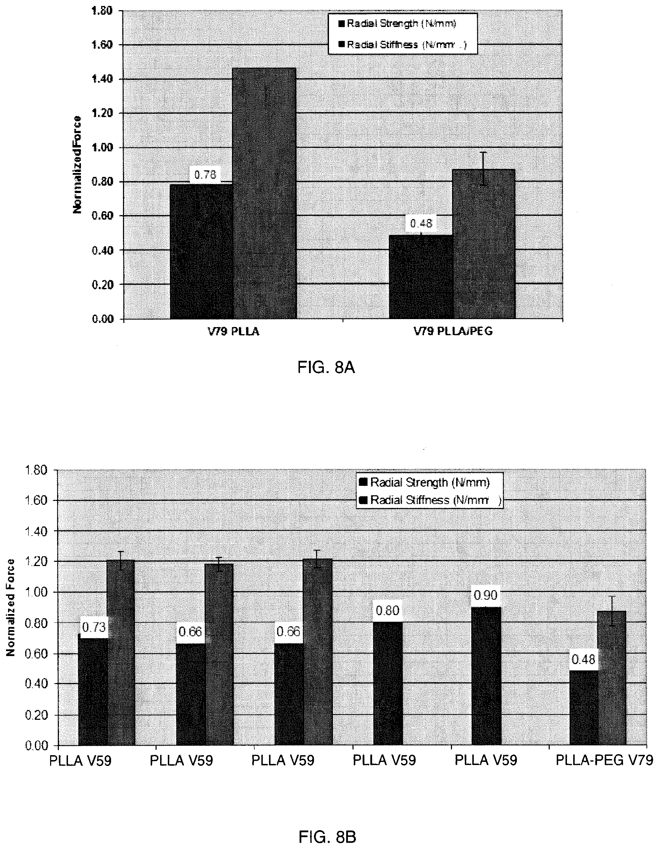

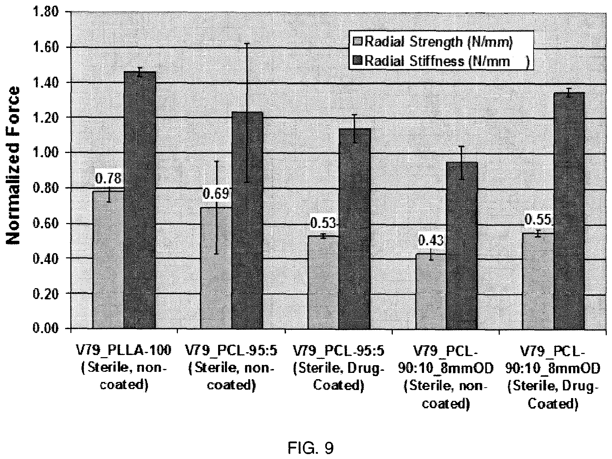

FIG. 8A shows the radial strength and stiffness for PLLA V79 and PLLA-PEG V79 scaffolds. FIG. 8B shows the radial strength and stiffness for several samples of PLLA V59 and PLLA-PEG V79 scaffolds. FIG. 9 shows the radial strength and stiffness for PLLA V79 scaffolds, 95:5 PLLA-PCL uncoated V79 scaffolds, 95:5 PLLA-PCL coated V79 scaffolds, 90:10 PLLA-PCL uncoated V79 scaffolds, and 90:10 PLLA-PCL uncoated V79 scaffolds.

### Crystallinity

The crystallinity of the polymer pellets used as feed to the extrusion, the extruded tube, the expanded tube, and the sterilized scaffold are given in Table 2.

**Table 2. Crystallinity of PLLA and PLLA-PEG-PLLA.**

| | PLLA | PLLA-PEG-PLLA |
|---|---|---|
| | % Crystallinity | |
| Pellets | 73 | 77 |
| Extruded Tube | ∼1 | ∼1 |
| Expanded Tube | 37 | 32 |
| Scaffold (sterilized) | 46 | 44 |

### Molecular Weight:

The number average molecular weights (Mn) of a PLLA and PLLA-PEG block copolymer scaffold were measured before and after e-beam sterilization and the results oare shown in Table 3

**Table 3. Number average molecular weights of PLLA and PLLA-PEG scaffolds.**

| Scaffold | Mn b/f sterilization (Da) | Mn after sterilization (Da) |
|---|---|---|
| PLLA scaffold | 385,331 | 169,201 |
| PLLA-PEG | 521,623 | 98,650 |

### Post-dilation to fracture:

The scaffold samples were dilated from a crimped diameter and the minimum OD at fracture was observed. Table 4 summarizes the results of the post-dilation to fracture results.

**Table 4. Post-Dilation to fracture of scaffolds**

| Scaffold Type | Post-Dilation to Fracture (mm) |
|---|---|
| PLLA V79 | 9.6mm |
| PLLA-PEG - V79 | 11.0 ± 0.8 mm |
| PLLA Lot 1 - V59 | 10.0 ± 0.6 mm |
| PLLA Lot 2 - V59 | 10.0 ± 0.1 mm |
| 95:5 PLLA-PCL - V79 | 10.0 ± 0.1 mm |
| 90:10 PLLA-PCL - V79 | 10.7 ± 0.8 mm |

### Crush Recovery:

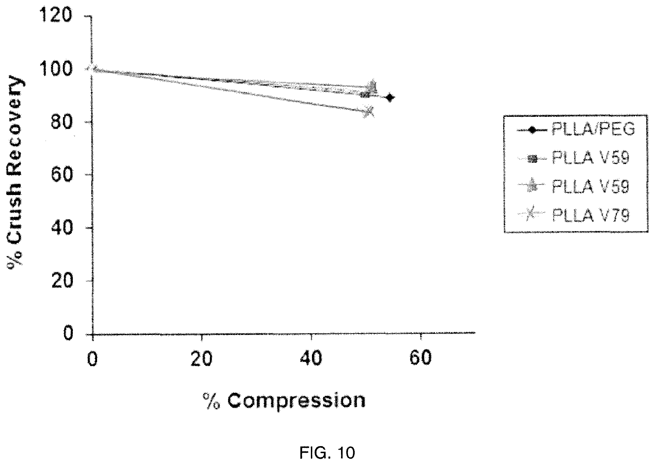

The crush recovery of two lots of V59 PLLA scaffolds, a V79 PLLA scaffold and a V79 PLLA-PEG scaffold was were tested and the results are shown in FIG. 10.

### Axial Fatigue:

The V79 PLLA, V59 PLLA, V79 PLLA-PEG, V79 95:5 PLLA-PCL, and V79 90:10 PLLA-PCL scaffolds were subjected to axial fatigue testing.

The scaffolds were coated with silicone sealant and deployed inside silicone tubing with two different hardnesses. The samples were dried for 24 hours and the tubes were stretched axially by 7%. The scaffold samples were then subjected to 500k cycles at 1Hz in circulating water at 37°C. The scaffold samples were inspected for fractures at different time points. Table 5 summarizes the data of the axial fatigue tests.

**Table 5. Axial fatigue data for V59 PLLA and V79 PLLA/PEG scaffolds.**

| | | Fracture Location | |
|---|---|---|---|
| Sample* | %Stretch | Ring | Link |
| PLLA/PEG. 40 DT, #1 | 3.9 | 0 | 0 |
| PLLA/PEG, 40 DT, #2 | 6.5 | 0 | 0 |
| PLLA/PEG, 40 DT, #3 | 2.7 | 0 | 0 |
| PLLA/PEG, 40 DT, #4 | 3.8 | 0 | 0 |
| PLLA/PEG, 40 DT, #5 | 3.8 | 0 | 0 |
| PLLA/PEG, 18 DT, #1 | 7.7 | 0 | 0 |
| PLLA/PEG, 18 DT, #2 | 7.0 | 0 | 0 |
| PLLA/PEG, 18 DT, #2 | 8.3 | 0 | 0 |
| PLLA/PEG, 18 DT, #2 | 6.3 | 0 | 0 |
| PL LA/PEG, 18 DT, #2 | 8.6 | 0 | 0 |
| PLLA, 40 DT, #1 | 1.9 | 0 | 0 |
| PLLA, 40 DT, #2 | 3.2 | 1 | 1 |
| PLLA, 40 DT, #3 | 3.2 | 2 | 3 |
| PL LA, 40 DT, #4 | 3.4 | 0 | 2 |
| PLLA, 18 DT, #1 | 5.5 | 5 | 2 |
| PLLA, 18 DT, #2 | 5.5 | 5 | 3 |
| PLLA, 18 DT, #3 | 5.5 | 3 | 2 |
| PLL A 18 DT, #4 | 5.5 | 10 | 2 |

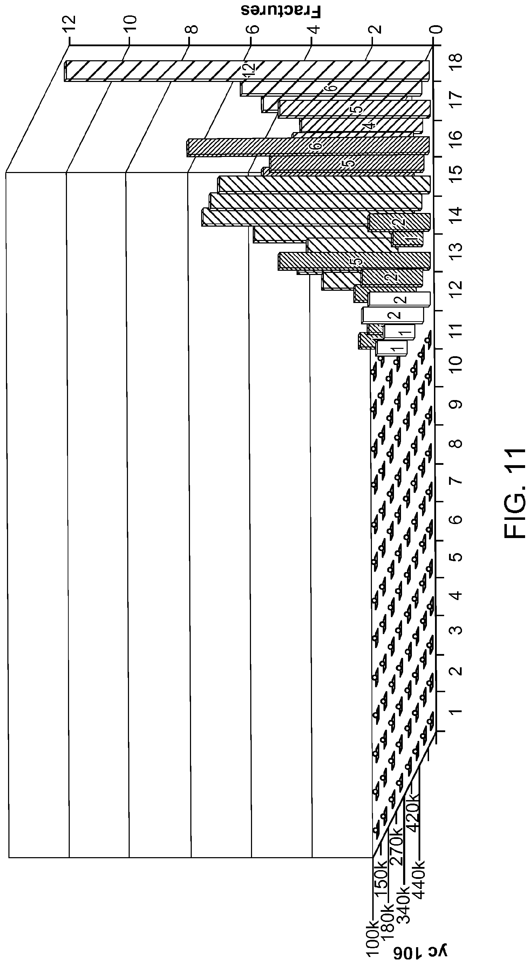

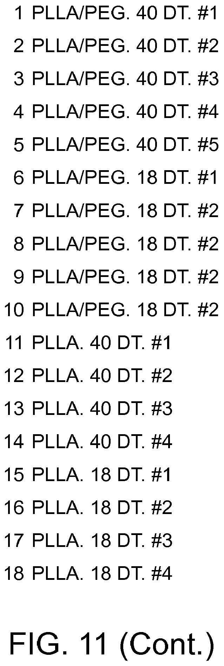

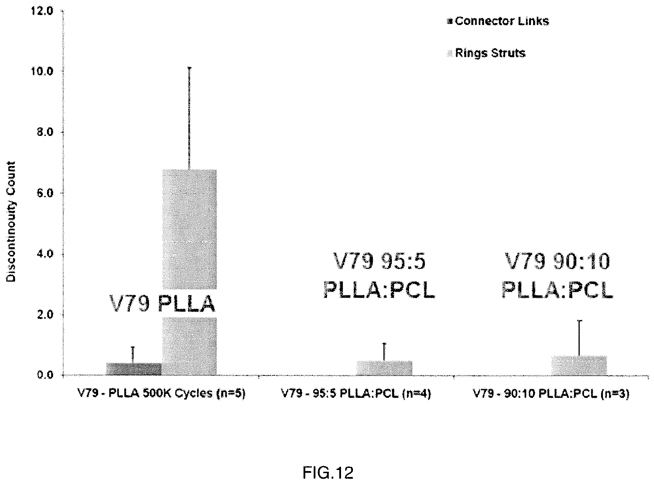

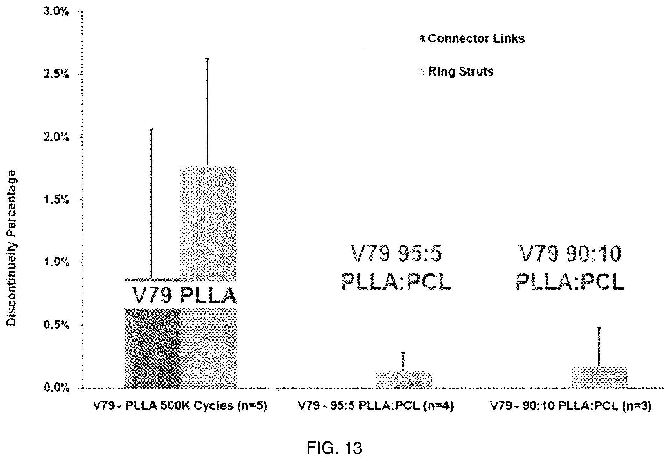

The PLLA-PEG scaffold samples had no fractures at 500k cycles. Other scaffolds showed fractures at 500k cycles. FIG. 11 depicts the number of cycles vs. the fracture for V59 PLLA and V79 PLLA-PEG scaffolds. FIG. 12 shows discontinuity count in ring and connector links after axial fatigue testing for the V79 PLLA, V79 95:5 PLLA-PCL, and V79 90:10 PLLA-PCL scaffolds. FIG. 13 shows discontinuity percentage in ring and connector links after axial fatigue testing for the V79 PLLA, V79 95:5 PLLA-PCL, and V79 90:10 PLLA-PCL scaffolds.

### Imaging of scaffolds post deployment

Images of the scaffolds post deployment were generated the Visicon Finescan™ Stent Inspection System, Visicon Inspection Technologies, LLC (Napa, Calif.). The system employs a scan camera to generate a flat, unrolled view of a scaffold. In operation, the scaffold is mounted on a mandrel with a fine diffuse surface. This mandrel is held under the linear array camera and rotated by the system electronics and is used to trigger the camera to collect a line of image data in a precise line-by-line manner.

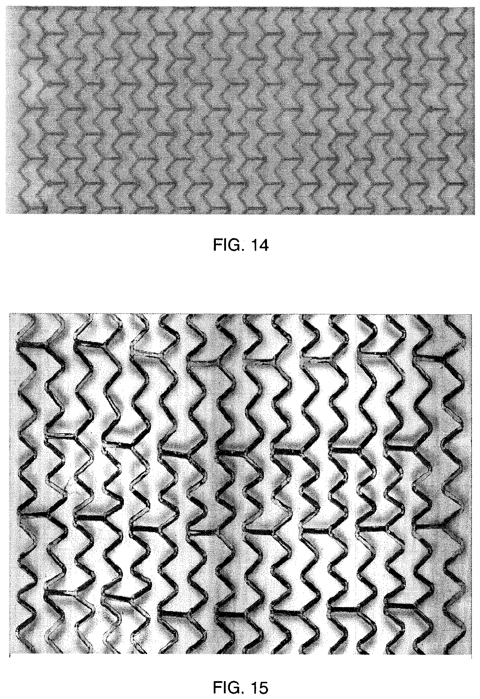

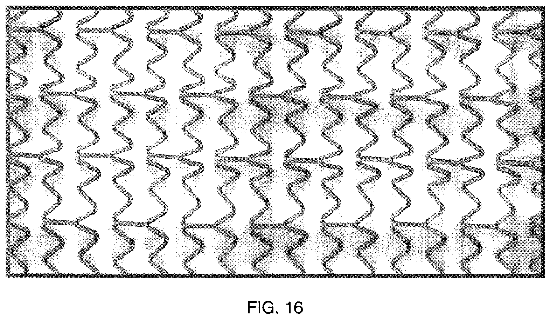

FIGs. 14-16 depict Finescan images of the V59 PLLA scaffold and the V79 PLLA and V79 PLLA-PEG scaffolds, respectively, post deployment.

## Claims

1. A stent comprising a scaffold formed from a polymer tube
- configured for being crimped to a balloon,
- the scaffold having a pattern of interconnected elements,
- the scaffold having an expanded diameter when expanded from a crimped state by the balloon,
wherein the scaffold attains greater than about 80% of the expanded diameter after being crushed to at least 50% of the expanded diameter; wherein the scaffold has a radial stiffness greater than 0.3 N/mm;
wherein the scaffold is made from a block copolymer of poly(L-lactide) (PLLA) and a hydrophilic polymer capable of having self expanding properties at 37 deg C; and
wherein the scaffold has rings formed by struts, and the struts form undulating rings having crowns and the rings are interconnected by longitudinally extending links, wherein the crowns include W-crowns, Y-crowns, and free crowns,
wherein a link connects a W-crown of a ring to a first adjacent ring and another link connects a Y-crown of the ring to a second adjacent ring, wherein a free crown is not connected to a link; and
wherein the rings comprise a sequence of crowns including: W-crown, 3 free crowns, Y-crown, 3 free crowns, and W-crown.

2. The stent of claim 1, wherein the hydrophilic polymer has a Tg less than 25 deg C.

3. The stent of claim 1, wherein the hydrophilic polymer is 0.1 to 10wt% of the block copolymer.

4. The stent of claim 1, wherein a glass transition temperature (Tg) of the block copolymer is between 37 deg C and 50 deg C in a wet physiological state.

5. The stent of claim 1, wherein a glass transition temperature (Tg) of the block copolymer is between 37 deg C and 50 deg C in a dry state.

6. The stent of claim 1, wherein hydrophilic polymer is polyethylene glycol (PEG).

7. The stent of claim 1, wherein the hydrophilic polymer is selected from the group consisting of polyethylene oxide (PEO), and polyvinylpyrrolidone (PVP), and polyvinyl alcohol (PVA).

8. The stent of claim 1, wherein a weight average molecular weight of hydrophilic block(s) of the block copolymer is 1 to 30 kDa.

9. The stent of claim 1, wherein the block copolymer is PLLA-b-PEG-b-PLLA.

10. The stent of claim 1, wherein the block copolymer is PLLA-b-PEG or PEG-b-PLLA- b-PEG.

11. The stent of Claim 1 , wherein the crowns have a maximum crown angle between about 90 degrees and 115 degrees when the scaffold has the expanded diameter.

12. The stent of Claim 1, wherein in an as-fabricated state:
the scaffolding has an outer diameter of 8 to 10 mm, the crown angles for rings of the scaffold are between 90 and 115 degrees, and the scaffold has a wall thickness of at least 0.2032 mm (0.008 in).

13. The stent of Claim 1, wherein the scaffold pattern includes a symmetric closed cell having a plurality of crowns, wherein any of the crowns have a maximum crown angle of between about 90 degrees and 115 degrees when the scaffold has the expanded diameter.

14. The stent of Claim 1, wherein the sequence of crowns further comprises 2 W-crowns and 2 Y-crowns.

## Patentansprüche

1. Ein Stent umfassend ein aus einem polymeren Rohr gebildetes Gerüst,
- das konfiguriert ist, um an einem Ballon gecrimpt zu werden,
- wobei das Gerüst einen Muster von vernetzten Elementen hat,
- wobei das Gerüst einen ausgedehnten Durchmesser hat, wenn es durch den Ballon aus einem gecrimpten Zustand ausgedehnt wird,
wobei das Gerüst mehr als ca. 80% des ausgedehnten Durchmessers erreicht, nachdem es bis zu mindestens 50% des ausgedehnten Durchmessers zusammengedrückt wird; wobei das Gerüst eine radiale Steifheit hat, die größer als 0,3 N/mm ist;
wobei das Gerüst aus einem Blockcopolymer von Poly(L-Lactid) (PLLA) und einem hydrophilen Polymer besteht, welches selbstexpandierende Eigenschaften bei 37 °C aufweisen kann; und
wobei das Gerüst durch Streben gebildete Ringe hat und die Streben wellige Ringe bilden, die Kronen aufweisen und die Ringe durch sich längst erstreckende Verbindungselemente vernetzt sind, wobei die Kronen W-Kronen, Y-Kronen und freie Kronen umfassen,
wobei ein Verbindungselement eine W-Krone eines Rings mit einem ersten angrenzenden Ring verbindet und
ein anderes Verbindungselement eine Y-Krone des Rings mit einem zweiten angrenzenden Ring verbindet, wobei eine freie Krone mit einem Verbindungselement nicht verbunden ist; und
wobei die Ringe eine Reihe von Kronen umfassend: W-Krone, 3 freie Kronen, Y-Krone, 3 freie Kronen und W-Krone umfassen.

2. Der Stent des Anspruchs 1, wobei das hydrophile Polymer eine Tg von weniger als 25 °C hat.

3. Der Stent des Anspruchs 1, wobei das hydrophile Polymer von 0,1 bis 10 Gew.-% des Blockcopolymers ausmacht.

4. Der Stent des Anspruchs 1, wobei eine Glasübergangstemperatur (Tg) des Blockcopolymers von zwischen 37 °C und 50 °C in einem feuchten physiologischen Zustand reicht.

5. Der Stent des Anspruchs 1, wobei eine Glasübergangstemperatur (Tg) des Blockcopolymers von zwischen 37 °C und 50 °C in einem trockenen Zustand reicht.

6. Der Stent des Anspruchs 1, wobei das hydrophile Polymer Polyethylenglycol (PEG) ist.

7. Der Stent des Anspruchs 1, wobei das hydrophile Polymer ausgewählt aus der Gruppe bestehend aus Polyethylenoxid (PEO) und Polyvinylpyrrolidon (PVP) und Polyvinylalkohol (PVA) ist.

8. Der Stent des Anspruchs 1, wobei ein Gewichtsmittel des Molekulargewichts des hydrophilen Blocks bzw. der hydrophilen Blöcke des Blockcopolymers 1 bis 30 kDa beträgt.

9. Der Stent des Anspruchs 1, wobei das Blockcopolymer PLLA-b-PEG-b-PLLA ist.

10. Der Stent des Anspruchs 1, wobei das Blockcopolymer PLLA-b-PEG oder PEG-b-PLLA- b-PEG ist.

11. Der Stent des Anspruchs 1, wobei die Kronen einen maximalen Kronenwinkel von zwischen ca. 90 Grad und 115 Grad haben, wenn das Gerüst den ausgedehnten Durchmesser aufweist.

12. Der Stent des Anspruchs 1, wobei es in einem wie-hergestellt-Zustand folgendes gilt:
das Gerüst einen Außendurchmesser von 8 bis 10 mm hat, die Kronenwinkel für Ringe des Gerüstes zwischen 90 und 115 Grad betragen, und das Gerüst eine Wandstärke von mindestens 0,2032 mm (0,008 in) hat.

13. Der Stent des Anspruchs 1, wobei der Gerüstmuster eine symmetrische geschlossene Zelle umfasst, welche eine Vielzahl von Kronen hat, wobei jedwede Krone einen maximalen Kronenwinkel von zwischen ca. 90 Grad und 115 Grad hat, wenn das Gerüst den ausgedehnten Durchmesser aufweist.

14. Der Stent des Anspruchs 1, wobei die Reihe von Kronen ferner 2 W-Kronen und 2 Y-Kronen umfasst.

## Revendications

1. Un stent comprenant un échafaudage formé à partir d'un tube polymère
- configuré pour être serti à un ballonnet,
- l'échafaudage ayant un motif d'éléments interconnectés,
- l'échafaudage ayant un diamètre expansé lorsqu'il est expansé à partir d'un état serti par le ballonnet,
dans lequel l'échafaudage atteint plus d'environ 80 % de son diamètre expansé après être écrasé jusqu'au moins 50 % du diamètre expansé ; dans lequel l'échafaudage a une rigidité radiale supérieure à 0,3 N/mm ;
dans lequel l'échafaudage est fait en un copolymère bloc de poly(L-lactide) (PLLA) et un polymère hydrophile qui peut avoir des propriétés d'autoexpansion à 37 °C ; et dans lequel l'échafaudage a des anneaux formés par des entretoises, et les entretoises forment des anneaux ondulés ayant des couronnes et les anneaux sont interconnectés par des éléments de liaison s'étendant longitudinalement, dans lequel les couronnes incluent des couronnes en W, des couronnes en Y, et des couronnes libres,
dans lequel un élément de liaison relie une couronne en W d'un anneau à un premier anneau adjacent et
un autre élément de liaison relie une couronne en Y de l'anneau à un second anneau adjacent, dans lequel une couronne libre n'est pas reliée à un élément de liaison ; et dans lequel les anneaux comprennent une séquence de couronnes incluant : couronne en W, 3 couronnes libres, couronne en Y, 3 couronnes libres, et couronne en W.

2. Le stent de la revendication 1, dans lequel le polymère hydrophile a une Tg inférieure à 25 °C.

3. Le stent de la revendication 1, dans lequel le polymère hydrophile constitue de 0,1 à 10 % en poids du copolymère bloc.

4. Le stent de la revendication 1, dans lequel une température de transition vitreuse (Tg) du copolymère bloc est d'entre 37 °C et 50 °C dans un état physiologique humide.

5. Le stent de la revendication 1, dans lequel une température de transition vitreuse (Tg) du copolymère bloc est d'entre 37 °C et 50 °C dans un état sec.

6. Le stent de la revendication 1, dans lequel le polymère hydrophile est le polyéthylène glycol (PEG).

7. Le stent de la revendication 1, dans lequel le polymère hydrophile est choisi dans le groupe constitué de l'oxyde de polyéthylène (PEO), et de la polyvinylpyrrolidone (PVP) et l'alcool polyvinylique (PVA).

8. Le stent de la revendication 1, dans lequel une masse moléculaire moyenne en poids du(des) bloc(s) hydrophile(s) du copolymère bloc est de 1 à 30 kDa.

9. Le stent de la revendication 1, dans lequel le copolymère bloc est le PLLA-b-PEG-b-PLLA.

10. Le stent de la revendication 1, dans lequel le copolymère bloc est le PLLA-b-PEG ou le PEG-b-PLLA- b-PEG.

11. Le stent de la revendication 1, dans lequel les couronnes ont un angle de couronne maximale d'entre environ 90 degrés et 115 degrés lorsque l'échafaudage a le diamètre expansé.

12. Le stent de la revendication 1, dans lequel dans un état tel que fabriqué :
l'échafaudage a un diamètre extérieur de 8 à 10 mm, les angles de couronne pour des anneaux de l'échafaudage sont d'entre 90 et 115 degrés, et l'échafaudage a une épaisseur de paroi d'au moins 0,2032 mm (0,008 in).

13. Le stent de la revendication 1, dans lequel le motif d'échafaudage inclut une cellule fermée symétrique ayant une pluralité de couronnes, dans lequel chaque couronne a un angle de couronne maximal d'entre environ 90 degrés et 115 degrés lorsque l'échafaudage a le diamètre expansé.

14. Le stent de la revendication 1, dans lequel la séquence de couronnes comprend en outre 2 couronnes en W et 2 couronnes en Y.
